# EUROPEAN PATENT APPLICATION

(11) **EP 1 417 958 A1**
(43) Date of publication of application: **12.05.2004**
(21) Application number: 02025006.4
(22) Date of filing: 08.11.2002
(51) Int. Cl.: A61K 9/16, A61K 31/427, A61K 31/472

(54) **Wet granulation process**

(71) Applicant: Pari GmbH, 82319 Starnberg (DE)
(72) Inventor: Lintz, Frank-Christophe, 82319 Starnberg (DE); Keller, Manfred, 79189 Bad Krozingen (DE)
(74) Representative: Albrecht, Thomas, Dr.

(57) **Abstract**

The invention is in the field of pharmaceutical dosage forms, and more particularly in the field of pharmaceutical granules and processes for making granules.

The invention provides a process for preparing pharmaceutical granules which contain an active ingredient in the form of a salt, said process comprising the steps of (a) providing a powder containing the active ingredient as a free base or acid, and (b) agglomerating the powder by adding a granulation liquid to form granules; wherein step (b) is conducted in the presence of a neutralization agent capable of neutralizing the active ingredient, and for a sufficient amount of time to allow the active ingredient to become at least partially converted into a salt.

The invention also provides pharmaceutical granules obtainable by said process and pharmaceutical compositions comprising said granules.

The invention further provides the use of pharmaceutical granules for the pulmonary delivery of an active ingredient.

## Description

The present invention is in the field of pharmaceutical dosage forms, and more particularly in the field of pharmaceutical granules and processes for making granules.

Pharmaceutical granules may be defined as preparations substantially consisting of solid, dry aggregates of powder particles sufficiently resistant to withstand handling. They typically contain one or more active ingredients, unless they constitute placebo granules. They can be administered as they are, but more commonly by first being sprinkled on food, or in the form of a solution or suspension after allowing the granules to disintegrate, and optionally dissolve, in an appropriate liquid carrier such as water. Granules are also used as intermediates for the preparation of other solid dosage forms, including tablets and hard capsules.

The rationale behind using granules for the preparation of liquid forms can vary depending on the specific product type and route of administration. One important use is that of administering drug compounds via the oral route in an easy and convenient way. It is natural for humans to swallow liquids and, from a certain age, also semi-solid materials. In contrast, the ingestion of solid materials that are larger than a few millimeters in diameter does not seem to be an essential capability of humans, and is probably most often practiced with medicines. It is not surprising that many patients, especially among children and elderly people, find it difficult to swallow tablets or capsules. Liquid pharmaceutical formulations for oral administration, on the other hand, are easy to swallow. Additionally, there may be other patients besides children and elderly people who find it also difficult to swallow tablets or capsules. The underlying reason may be pathological, such as for patients with impaired esophagus function, or it may be more or less psychological. Another aspect is the administration of large drug doses, whose incorporation requires very large tablets or capsules, which are difficult to swallow even for normal adult patients.

Besides the peroral administration route, liquid dosage forms are suggested for pulmonary drug delivery. Today, a large number of pharmaceutical products for inhalation are marketed either as pressurized metered dose inhalers (MDIs) or dry powder inhalers (DPIs). However, both technologies exhibit pharmaceutical problems and disadvantages. For instance, their ability in delivering a drug to the lungs relies on the patient's inspiration activity to disperse a powder formulation. Thus, they require a substantial air flow, such as about 30 l/min, for effective pulmonary delivery. Many patients with impaired breathing function, such as asthmatic children or elderly people, are therefore not able to use these devices. Especially for these patients, nebulizers may be more useful to administer drugs via the pulmonary route. Pharmaceutical nebulizers produce inhalable aerosols from aqueous-based liquid formulations.

Despite all the advantages related to patient convenience and compliance mentioned above, liquid pharmaceutical formulations for peroral or pulmonary administration do not possess the typical advantages of solid dosage forms, such as dosing precision, physicochemical stability, practicability of packaging formats, and low manufacturing and storage costs. Additionally, the majority of pharmaceutical compounds for peroral or pulmonary drug delivery is not available as aqueous solution due to their poor solubility and insufficient chemical stability in aqueous media to allow for an acceptable shelf life. To overcome these drawbacks, attempts have been made to develop rapidly disintegrating solid dosage forms as intermediates for the preparation of liquid formulations upon dissolution in aqueous vehicle.

Effervescent tablets have been proposed. However, they are relatively complex and expensive to produce since they require special equipment and suitable premises for processing powders with a controlled moisture content. Furthermore, chemical stability issues of the drug substance in the presence of acids have to be considered. Additionally, due to their high fragility, friability and moisture sensitivity, these products are difficult to handle and require specific packaging. For the very same reasons and because of the high manufacturing costs, lyophilized products are not suggested as intermediate dosage form of first choice for the preparation of liquid formulations.

In WO 95/07070, an effervescent granulated material and a method for its preparation is described. The effervescent granules contain citric acid and calcium carbonate, or similar effervescent components, in a partially reacted state. The granules are useful for oral administration, and for the preparation of effervescent tablets, but not for the preparation of solutions for inhalation.

US 6,149,938 discloses a process for the preparation of a granulate suitable for the preparation of rapidly disintegratable mouth-soluble tablets. The process involves the granulation of a powder substrate in a fluidized bed, and the incorporation of an edible acid which is not a member of an effervescent couple. Even though the resulting granules and the tablets made therefrom disintegrate rapidly, there is no hint at the role of the edible acid and its influence on the product characteristics aside from palatability. Furthermore, the process is not useful for the preparation of granules for other than oral delivery purposes because it requires the incorporation of polyvinyl alcohol and/or other polymers.

US 6,014,970 discloses the use of a solid, drug substance-containing material for the *in-situ* generation of a liquid aerosol within an aerosolizer. However, there is no disclosure on how to prepare such material which, upon dissolution, leads to a physiologically acceptable solution for inhalation, neither is it disclosed how a poorly soluble acidic or basic drug incorporated in the material can be dissolved rapidly and efficiently.

Usteri et al. (Acta Pharm. Technol. 35 (3) 163-167, 1989) describe the granulation with surfactants, and teach the incorporation of surfactants into granules in order to achieve an enhanced dissolution of poorly soluble drug substances. However, the surfactants that are suggested are not suitable for all routes of administration, and the achievable dissolution rates may not be sufficient for poorly soluble acidic or basic drug substances.

Thus, there is a need for a process for the preparation of solid dosage forms which can be administered as such, or easily and rapidly dissolved in aqueous media to prepare an aqueous solution or suspension. Furthermore, there is a particular need for rapidly soluble compositions containing poorly soluble drug substances.

It is therefore an object of the invention to provide a method for manufacture of a rapidly disintegrating solid dosage form as intermediate for preparing a liquid dosage form, the solid dosage form containing an active compound with enhanced water solubility. It is another object of the invention to provide a solid dosage form as intermediate which is easy to handle, and which yields a liquid dosage form with improved tolerability when administered perorally or by inhalation.

It is another object of the invention to provide a process for the preparation of pharmaceutical granules, said granules comprising a poorly soluble drug substance. Yet another object of the invention is to provide a process for the preparation of pharmaceutical granules, said granules being dissolvable in an aqueous carrier to form a solution having a physiologically acceptable pH value.

### SUMMARY OF THE INVENTION

In a first aspect, the invention provides a process for preparing pharmaceutical granules containing an active ingredient as a salt. The process comprises the steps of (a) providing a powder containing the active ingredient as a free base or free acid, and (b) agglomerating the powder by adding a granulation liquid to form granules. Step (b) of the process is conducted in the presence of a neutralization agent which is capable of neutralizing the active agent, and for a sufficient amount of time to allow the active ingredient to be at least partially converted into a salt. The process is particularly useful for the preparation of granules containing an active ingredient whose water solubility as a free base or free acid is low. The resulting granules comprise the active ingredient as a salt, typically with improved solubility. The granules are prepared with high efficiency as the process combines the neutralization of the drug substance to form a salt and the agglomeration of the powder to form a granular material in a single step.

In a second aspect, the invention provides pharmaceutical granules which are obtainable by the process of the invention. The granules comprise an active ingredient which was at least partially converted from a free base or acid into a salt. Typically, the granules disintegrate rapidly and allow the fast dissolution of the drug substance contained therein.

In a further aspect, pharmaceutical compositions are provided which comprise the granules prepared by the process of the invention, or which are prepared by compressing said granules. The compositions may comprise further excipients. They are particularly useful for the administration of an active ingredient which, as a free acid or base, is poorly soluble or unstable.

In a further aspect, the invention provides uses of pharmaceutical granules, including the granules that are prepared by the process of the invention, and of pharmaceutical compositions comprising such granules. The uses include the administration of poorly soluble drugs and the delivery of active compounds via the pulmonary route.

Further aspects and embodiments of the invention will become clear through the detailed description, the examples, and the claims as set forth below.

### DETAILED DESCRIPTION OF THE INVENTION

The process of the invention leads to pharmaceutical granules which contain an active ingredient in the form of a salt, and it comprises the steps of (a) providing a powder containing the active ingredient as a free base or free acid, and (b) agglomerating the powder by adding a granulation liquid to form granules. Step (b) of the process is conducted in the presence of a neutralization agent which is capable of neutralizing the active agent, and for a sufficient amount of time to allow the active ingredient to be at least partially converted into a salt.

Pharmaceutical granules may be defined as preparations substantially consisting of solid, dry aggregates of powder particles sufficiently resistant to withstand handling. They typically contain one or more active ingredients, unless they constitute placebo granules. They can be administered as they are, but more commonly they are administered by first being sprinkled on food, or in form of a solution or suspension after allowing the granules to disintegrate, and optionally dissolve, in an appropriate liquid carrier such as water. Granules are also used as intermediates for the preparation of other solid dosage forms, including tablets and hard capsules.

An advantage of granules over powders is that they can be handled much better. They have improved flowing characteristics and produce little dust. Thus, granules can be more easily dosed, both within a tabletting or capsule-filling process and by the dispensing pharmacist or patient prior to administration. Another important aspect of granules as intermediates in the manufacture of tablets is their superior compressibility.

An active ingredient, or drug substance, may be defined as any compound or mixture of compounds that may be used to prevent, treat, diagnose, or predict a disease or undesirable condition, or a risk factor thereof. A majority of the presently known active ingredients are chemical or biological substances which possess at least one group or atom which may be ionized, and which can participate in the formation of a base or acid addition salt. In some cases, drug substances are available both as a free base or acid and as a salt; in other cases, only a salt or a free base or acid is commercially available. In the process of the invention, an active ingredient is used which is available as a free base or acid and provided as such. During the process, however, it is partially or completely converted into a salt by means of a neutralizing agent.

The process is characterized in that it comprises at least two steps, these steps being (a) providing a powder containing the active ingredient as a free base or free acid, and (b) agglomerating the powder by adding a granulation liquid to form granules. Step (b) is conducted after step (a), but not necessarily in an immediate succession. This second step of the process is based on a common method for preparing granules, which is often referred to as wet granulation.

In step (a), a powder is provided which is then agglomerated in step (b). A powder may be defined as a preparation consisting of solid, loose, dry particles of varying degrees of fineness. According to the invention, the powder comprises at least one active compound. In principle, powders can be agglomerated to granules by various methods. These methods may be categorized as wet granulation methods and dry granulation methods. According to the invention, only wet granulation methods can be used. These methods have in common that a suitable granulation liquid is added to the powder to agglomerate it. The granulation liquid is needed for agglomeration because it forms physical or mechanical bonds between powder particles. The bonds may consist of a binder which is provided with the liquid. The binder solidifies and forms bridges between individual powder particles as the solvent of the granulation liquid evaporates. Alternatively, the liquid may be free of binders but capable of at least partially dissolving at least one of the powder constituents. After the removal of the solvent, solid bridges between powder particles are formed from the dissolved constituent.

In the process of the invention, the agglomeration of the powder by adding a granulation liquid is conducted in the presence of a neutralization agent capable of neutralizing the active ingredient which was provided as a free base or acid. As used herein, the neutralization agent is any base, acid, ion, or salt which can form a salt with the active ingredient. In this sense, the terms "neutralizing", "neutralization", and "neutralize" are defined in a broad way to also include the conversion of the drug substance into a salt which may itself also be acidic or basic instead of absolutely neutral. The neutralization agent may be provided as a component of the granulation liquid, or of the powder.

In order to convert at least some of the active substance into a salt, the agglomeration step must be carried out for a sufficient amount of time. The actual amount of time needed may be easily determined experimentally by a person skilled in the art; it will strongly depend on the physicochemical characteristics of the drug substance, the neutralization agent, the rate in which the granulation liquid is added, the intensity of agitation, the desired degree of neutralization, and several other factors.

Rather typically, the duration of this step may be in the range of only one minute or less up to approximately one hour; only in few cases, longer agglomeration times will be needed. Many granulation methods using the process of the invention will be optimizable to allow an agglomeration time of one to about 15 minutes.

In one embodiment, step (b), i.e. the agglomeration of the powder by the addition of a granulation liquid in the presence of a neutralization agent, is carried out for a sufficient amount of time to allow the active ingredient to become completely or almost completely converted into a salt. This embodiment is particularly useful when the active ingredient, as a base or acid, is a poorly soluble compound. Especially when such a poorly soluble drug substance needs to be formulated into granules which dissolve in a small amount of liquid, or when it is desirable that the drug substance dissolves rapidly, it is advisable to conduct the process in such a way that substantially all of the drug is converted into a salt which is better soluble than the free acid or base. It is therefore preferred that the drug, in the form of the free acid or base in which it is provided, has a limited water solubility at room temperature (20 °C), such as less than about 10 % by weight. More preferably, the water solubility is less than about 5 %. In other preferred embodiments, the drug substance is sparingly soluble, slightly soluble, very slightly soluble, or practically insoluble as commonly defined in the pharmaceutical sciences. In a similar fashion, the drug substance may be poorly soluble in a physiological fluid, such as gastric or intestinal fluid.

As a result of the process of the invention, granules are formed which contain the active ingredient as a salt. Typically, the neutralization agent will be selected to convert the free base or acid of the drug substance into a salt which is substantially better soluble in water or in a physiological fluid of interest. Preferably, the solubility of the salt at room temperature (20 °C) is at least about 1 % by weight. Even more preferred are embodiments in which the solubility of the salt is considerably higher, such as at least about 3 %, or at least 5 %, or even 10 % or more. The advantage of converting the drug into a better soluble salt can also be measured by the relative improvement in solubility. For instance, for a practically insoluble drug base the process may be useful even when it leads to granules comprising a drug salt with a solubility of only 1 wt.-%. In other words, it is preferred that the resulting salt has a solubility which is substantially higher, such as at least 20 % higher, than the drug substance as provided. The advantage is of course greater when the solubility of the salt is much higher in relation to that of the free base or acid, such as at least twice or three times as high, or even more than ten times as high.

The drug substance itself may be any active ingredient which is a base or an acid, and may be useful in any therapeutic indication. Examples include beta-agonists, such as salbutamol, levalbuterol, formoterol, fenoterol, salmeterol, bambuterol, brocaterol; anticholinergics, such as tiotropium, oxitropium, ipratropium; local anaesthetics, such as lidocaine, procaine; mucolytics, such as cysteine; anti-inflammatory drugs comprising mediator cell inhibitors, such as cromoglycinic acid, lidocaine; corticosteroids, such as beclomethasone, triamcinolone; antibiotics, including beta-lactam antibiotics, such as amoxicillin, piperacillin, clavulan acid; cephalosporines, e.g. ceftibuten, cefoxitin; monobactames, e.g aztrenonam; aminoglycosides, such as streptomycin, neomycin, colistin, kanamycin, gentamycin, amicacin, tobramycin; tetracyclines, such as doxycycline; polyene antibiotics, such as amphotericine B; drugs to treat pulmonary hypertension, such as phosphodiesterase inhibitors including sildenafil, vardenafil; antimetabolites, e.g. fluorouracil; alkaloids, such as papaverine, vinblastine, vincristine, vindesine; antibiotics, such as alcarubicine, bleomycine, dactinomycine, doxorubicine, mitomycine; anti-migraine drugs, such as barbituric acids derivatives, lisuride, methysergide, ergotamine, clonidine; hypnotics, sedatives, benzodiazepines, barbituric acid and its derivatives, phenytoin, valproic acid; antiparkinson drugs, such as levodopa, carbidopa, selegiline, bromocriptine, amantadine; antiemetics, such as thiethylperazine, bromopride, domperidone, ondansetrone, tropisetrone, pyridoxine; analgesics, such as morphine, codeine; drugs for narcosis, such as N-methylated barbituric acids, ketamine, etomidate, benzodiazepines, droperidol, haloperidol, alfentanyl, sulfentanyl; antirheumatism drugs including nonsteroidal antiinflammatory drugs such as salicylic acid. Of all mentioned active compounds, also their derivatives, conjugates, isomers, epimers, diastereomers, or racemic mixtures may be used.

The process of the invention is particularly suitable for poorly soluble drug compounds for the treatment of any disease or condition. Therefore, in one of the preferred embodiments, the active compound is useful in the therapy of conditions affecting the respiratory system, such as adult respiratory distress syndrome, bronchitis, asthma, chronic obstructive pulmonary disease, allergies, bacterial or viral infections.

Furthermore, the particularly preferred drug substances include salbutamol, levalbuterol, formoterol, fenoterol, salmeterol, bambuterol, brocaterol, tiotropium, oxitropium, ipratropium, lidocaine, procaine, cystein, cromoglycinic acid, beclomethasone, triamcinolone, amoxicillin, ceftibuten, cefoxitin, aztrenonam, colistin, tobramycin, doxycycline, sildenafil, vardenafil, barbituric acid derivatives, benzodiazepines, morphine, codeine, salicylic acid, including their derivatives, conjugates, isomers, epimers, diastereomers, or racemic mixtures.

While, in theory, the granulation liquid may comprise any solvent including water and organic solvents, it is preferred for toxicological and environmental reasons to use only physiologically tolerable liquids. Clearly, water is one of the most preferred solvents to be used in the process, optionally mixed with a water-miscible, more easily vaporizable organic solvent such as ethanol or acetone. Alternatively, the liquid may be substantially organic instead of aqueous-based, and contain one or more low molecular weight alcohols, in particular primary, secondary and tertiary C1 - C7 alcohols and alkanediols, such as ethanol, methanol, propanol, isopropanol, glycerol, polyethylene glycol and propylene glycol, and mixtures thereof. Non-alcoholic solvents include acetone, methylene chloride, and ethyl acetate.

The neutralization of the active compound and its conversion into a salt is achieved during the agglomeration step in which the powder is wetted by the granulation liquid: this step is conducted in the presence of the neutralization agent. Accordingly, the neutralization agent must be provided as a component of the powder, or as a component of the granulation liquid, or separately but simultaneously. As defined above, the neutralization agent is any base, acid, ion, or salt which is capable of forming a salt with the active ingredient.

Pharmaceutically acceptable acids, acidic salts, or anions which can form a salt with a drug substance which is provided as a free base include the following, non-limiting examples: citric acid, sulphuric acid, tartaric acid, malic acid, fumaric acid, adipic acid, succinic acid, alginic acid, lactic acid, gluconic acid, hydrochloric acid, phosphoric acid, fatty acids, fumaric acid, amino acids, acidic ammonium salts, benzoic acid, acid anhydrides, acidic metal salts such as disodium dihydrogen phosphate, dipotassium dihydrogen phosphate, and the corresponding citric acid salts. Among the preferred acidic neutralizing agents are citric acid, lysine, methionine, arginine, hydrochloric acid, and phosphoric acid.

Pharmaceutically acceptable bases, basic salts or cations which can form a salt with a drug substance which is provided as a free acid include the following, non-limiting examples: ammonium, alkali metal hydroxides, alkaline earth metal hydroxides, including sodium hydroxide, magnesium hydroxide, calcium hydroxide and amines, carbonates such as sodium carbonate, sodium bicarbonate, potassium carbonate, potassium bicarbonate, magnesium carbonate, and the mixtures thereof. Also suitable as base sources are amino acids, such as lysine, their carbonate salts, such as sodium glycine carbonate, L-lysine carbonate, arginine carbonate.

When provided with the granulation liquid, the neutralizing agent is preferably in the dissolved state to ensure a rapid and controlled neutralization process. When provided with the powder, the neutralizing agent is preferably a compound which dissolves rapidly in the granulation liquid. The amount of neutralization agent and duration of granulation are sufficient for a complete neutralization of the active compound provided in the powder. The preferred amount of neutralizing agent in the granulation liquid corresponds at least to the stoichiometric amount of the acidic or basic active component to be neutralized.

The process according to the invention is a wet granulation method. Wet granulation proceeds by a number of mechanisms for agglomerate growth and disintegration, the roles of which are dependent on the granulation equipment as well as the properties of the feed material, especially its particle size distribution. Agglomerate formation and growth of these powders can be described by the two growth mechanisms: nucleation of particles and coalescence between agglomerates.

Like other wet-granulation methods, the process of the invention can be carried out in any type of equipment adapted for the agitation of powders, and the wetting of powders with liquids. Common types of equipment which may be used for medium- and large-scale production include mixers, high-shear mixers, fluid-bed granulators, and rotary granulators. In one of the embodiments, the process of the invention comprises the addition of the granulation liquid to the powder from which the granules are to be formed by spraying the liquid through a nozzle onto the powder. Several types of granulation machines are equipped to allow this method, including most rotary granulators, high-shear mixers, and top-spray fluid-bed granulators.

No specific type of granulator is required for carrying out the process of the invention. The exact way in which the process is carried out may vary according to the needs of each individual case. For instance, if any of the starting materials and/or products are hygroscopic or reactive, the process environment may be rendered inert with nitrogen or carbon dioxide gas before or during the granulation process. In rotary granulators and high-shear mixers, the rate of the mixing tool and, optionally, that of the chopper will significantly influence the quality of the product. Furthermore, the process can be carried out either under atmospheric pressure or under reduced pressure. The process temperature may be room temperature (20 °C), or it may be lower for sensitive ingredients, or it may be elevated to 40°C or even up to about 110°C to speed up the process of removing volatile materials.

As an option, the granules formed in step (b) can be dried immediately after their formation. If the granulation equipment is appropriate for this purpose, the drying step can be carried out without interruption or product transfer. An example for such type of equipment is the fluid-bed granulator. In general, however, the drying of the granules can be conducted using hot air, microwaves or infra red light, under inert air conditions in the presence of nitrogen or carbon dioxide. Dried granules can be further processed to pellets by spheronization or to tablets by compression, as will be described later.

An important aspect of the invention are the granules themselves that are formed in the process as described above. These granules are agglomerates with a weight-average mean diameter of about 0.1 to 1.0 mm, and more preferably of 100 and 800 µm. Other useful mean diameters are between about 150 and 600 µm. The granules contain at least one active ingredient which was provided as a free base or acid, and which preferably has a low water solubility as defined above, and which was at least partially neutralized to form a salt with neutralization agent during the agglomeration step of the process. The granules thus contain at least some of said drug substance, and more preferably substantially all of said drug substance, in the form of its salt with the neutralization agent.

The granules may further contain an amount of unreacted neutralization agent. They may contain one or more further active ingredients, and they are very likely to contain one or more further inactive ingredients, or excipients.

Beyond the requirements of the process of the invention as such, the selection of further excipients is done according common pharmaceutical practice. In general, pharmaceutical excipients which may be used include bulking agents, fillers, binders, surfactants, stabilizers, buffers, preservatives, antioxidants, disintegrants, coloring agents, taste masking agents, sweeteners, flavors, release modifiers, plasticizers, glidants, and compression aids. The selection will strongly depend not only on the type and amount of active ingredient, but also to the intended use of the granules. For instance, if the granules are intended to be used for oral administration, a much larger selection of excipients may be used for their formulation than if they are intended for pulmonary administration.

In many known types of granules, but also in some granules according to the invention, binders are used. Appropriate binders for granules which are intended for oral use are known to the person skilled in the art. Common binders include gelatin, starch and starch derivatives, polyvinylpyrrolidone, cellulose ethers, natural and semisynthetic gums etc. In most cases, binders are added to the granulation liquid instead of being provided with the powder mixture. The presence of binders in the granulation liquid is efficient for the agglomerate formation and growth.

However, if the granules are intended for pulmonary administration, such as after their dissolution to form a solution for inhalation, it is preferred for reasons of physiological tolerability that they do not comprise any of the common polymeric binders. In fact, it is preferred that, in this case, they do not contain any polymeric excipients at all, which means that the granules have to be formed without the use of excipients which are very common in granules for oral use, such as polymeric binders and polymeric disintegrant. In another preferred embodiment, the granules do not contain any insoluble or practically insoluble materials, such as magnesium stearate and crosslinked polymers.

As alternative binders which are highly soluble, polyalcohols may be used. Preferred polyalcohols to be used as binding agents are selected from those commonly found in foods, and are preferably selected from the group of sugars or sugar alcohols with a water solubility at room temperature (20 °C) of at least 10 wt.-%, such as mannitol, xylitol, sorbitol, maltitol, erythritol, and lactitol. These excipients do not only function as binders; they may also accelerate the disintegration of the granules in aqueous media and the dissolution of the active ingredient. As the typical drug substance that is incorporated into granules according to the invention is a poorly soluble acid or base, it may be very desirable to use one of these dissolution-enhancing excipients. In one embodiment, the granules contain at least 10 % (w/w) of such an excipient with high water solubility. In another embodiment, the granules comprise at least about 25 % of such an excipient.

In another embodiment, all excipients, and thereby the granules themselves, are soluble in water or in an aqueous carrier, such as in a buffer solution. Such granules may be adapted for the pulmonary use. The aqueous carrier in which the granules may be dissolved is preferably a physiologically acceptable aqueous vehicle, and the resulting solution may be suitable for inhalation, such as with the aid of an ultrasonic or jet nebulizer.

The dissolution time is preferably short, such as less than about 2 minutes. If the granules are intended to be used for the preparation of a solution for inhalation, the dissolution time should be even shorter, and preferably less than about 1 minute, and more preferably less than about 30 seconds at room temperature (20 °C) in the physiologically acceptable aqueous vehicle or carrier. Such short dissolution times contribute to the accurate and reproducible dosing of the drug substance.

In other embodiments, various other excipients may be incorporated into the granules, especially if they are not intended for pulmonary drug delivery.

Bulking agents and fillers are typically chemically inert and physiologically tolerated excipients, which are utilized to increase the volume of the provided powder or granules and enable a homogeneous distribution of the active compound in the powder, in particular in case of a low drug substance concentration. Bulking agents and fillers may, for instance, be selected from the group of sugar alcohols, such as mannitol and lactitol. The preferred content of bulking agents and fillers ranges from 0 % (whenever not needed) to about 99.9 % (W/W) (for highly active drug substances).

Especially for granules incorporating a poorly soluble active ingredient, it may be desirable to also use one or more surfactants to enhance the dissolution of the drug substance. Such surfactants are selected from physiologically acceptable surfactants, and - especially if the granules are to be used for pulmonary delivery - more preferably from the group consisting of tyloxapol, polysorbates, phospholipids, and vitamin E-TPGS. The surfactant content in the granules is selected according to physiological consideration, but it is also related to the content and the physicochemical properties of the drug substance. For instance, a typical range of surfactant-to-drug ratio may be from about 1:100 to about 2:1. A more preferred range is from about 1:50 to about 1:5.

In other cases, the granules may comprise one or more compression aids. Compression aiding agents are chemically inert and physiologically tolerated excipients which can be incorporated into the granulate either during the granulation or during the final mixing phase before the compression of the granules to tablets. Compression aiding agents comprise mainly lubricants, which are suitable for improving the flow properties of the granules or final tabletting mixture and decreasing the friction of granules on the tabletting tools. Among the traditional solid lubricants, Ca, Mg, Zn salts of stearic acid, partially hydrogenated vegetable oils, polyethylene glycols, polyoxyethylene monostearate, talc, sodium benzoate, sodium laurylsulfate, and magnesium oxide can be used. The amount of lubricant to be added can vary from 0.1 % to 1.0% of the total composition. Adsorbing agents, such as colloidal silicon dioxide or talc, can also be understood as compression aiding agents. They are used as carriers of liquid components (e.g. flavors, active compound) or as adsorbent of moisture. As mentioned above, these excipients should be selected with regard to the intended use of the granules.

One or more flavors, which may be important components of perorally administered pharmaceutical granules, may be selected from commonly used synthetic or natural flavors in solid or liquid state alone or in a combination, such as plants extracts, essential oils including cinnamon, peppermint, clove, anise, eucalyptus, thyme, cedar, or chamomile oils, fruits essences including apple, peach, strawberry, raspberry, orange, apricot, cherry, plum, and pineapple. The amount of flavor used can vary depending on a number of factors such as the intensity of the desired organoleptic effect. A typical flavor content may range from 0.05 to 3.0% (w/w) of the granules, or of the pharmaceutical composition comprising the granules.

The addition of flavors very often leads to an insufficient taste masking, especially when the drug substance possesses a very strong bitter or metallic taste. In these cases, the formation of inclusion complexes of the active compound with a cyclodextrin such as beta-cyclodextrin or hydroxypropyl-beta-cyclodextrin, or the addition of any other taste masking additive know to the pharmaceutical formulation expert may have a positive impact on the palatability of the formulation. This may be especially relevant for granules which are to be administered orally, but also to those which are used for the preparation of a solution for inhalation, because even in this route of administration the bad taste of a drug substance can be very unpleasant to the patients.

The granules optionally contain dies and/or coloring agents such as titanium dioxide, acceptable natural or synthetic coloring agents, which may, for instance, be provided as a component of the granulation liquid and sprayed on the powder during the agglomeration step.

It is a further option to add stabilizing agents to the granules via the granulation liquid or via the powder. Stabilizers may include excipients such as antioxidants and pH-adjusting substances such as acids, bases, or buffer salts. Most often, these components are used in order to avoid or decrease the rate of drug substance degradation. Antioxidants can be divided in two groups: the free radical capturing agents, such as tocopherole, butylhydroxyanisole, butylhydroxytoluene, and easily oxidizable substances, such as ascorbic acid, its fatty acid esters, sulfite and hydrogen sulfite, which will be oxidized faster than the drug substance. In other words, they indirectly shield the active compound against oxidative degradation.

The invention further comprises compositions which are formulated from the granules described above. A pharmaceutical composition, taking the form of any appropriate pharmaceutical dosage form, may be represented by the granules themselves, or it may be formulated using the granules as a component, optionally along with further pharmaceutical excipients, into another dosage form, such as a hard capsule or a tablet. Common excipients which may be added to the granules include the same classes of additives described above, and may include bulking agents, fillers, binders, surfactants, stabilizers, buffers, preservatives, antioxidants, disintegrants, coloring agents, taste masking agents, sweeteners, flavors, release modifiers including excipients for delayed, sustained or controlled release, plasticizers, glidants, and compression aids. Again, the selection will strongly depend not only on the type and amount of active ingredient present in the granules, but also on the intended use of the composition. For instance, if the composition is intended to be used for oral administration, a much larger selection of excipients may be used for its formulation than if it is intended for pulmonary administration.

As mentioned, the compositions containing the granules can be provided in various different types of dosage forms, depending on the specific product application. In many cases, even these compositions, whether in granular, tablet, or capsule format, will not represent the administration form. The invention is particularly useful for the preparation of granules and/or compositions made thereof which are intended to be administered after their dissolution in an appropriate aqueous carrier. This liquid may be provided with the solid composition as a component of a product kit. Using the teachings of the invention, granules and/or compositions may be prepared from poorly soluble drug substance which are at least partially neutralized into a better soluble salt during the granulation process. Their enhanced dissolution in the aqueous carrier makes them particularly good candidates for solid formulations which are intended to be used for the preparation of a solution for inhalation, or for parenteral injection. For this purpose, the compositions should be substantially free of polymeric materials, such as common polymeric binders and disintegrants, and they should also be substantially free of insoluble excipients including some of the common insoluble powder lubricants.

For pulmonary administration, a liquid composition can be inhaled either through the nose or, more preferably, through the mouth. This is done, for instance, after nebulizing the liquid to form an aerosol, which is a dispersion of finely divided liquid droplets in a gaseous phase. Various nebulizers are known and available for pharmaceutical applications. They make use of several methods of nebulization, such as air jet nebulization, ultrasonication, shear forces generated at multiple apertures (vibrating membrane technology), or electrohydrodynamic activation by an ionized electric field. The liquid itself can be prepared prior to its use due dissolution of the intermediate solid composition as described in the invention.

The granules prepared by the process of the invention are particularly useful for pulmonary administration because, even though they comprise an acidic or basic drug substance, they lead to a physiologically acceptable solution for inhalation, having a relatively neutral pH. Furthermore, they can be prepared without the use of insoluble excipients such as binders, glidants, or disintegrants. The same advantages make them useful for parenteral administration after their reconstitution in an aqueous carrier.

However, liquid solutions or suspensions obtained by the dissolution or disintegration of the granules, or the solid compositions made therefrom, may also be used for other types of administration, including oral administration. This may be especially appropriate for the administration of drug substances in large single doses which are not easily swallowed as a solid single unit such as a capsule or tablet. Also, there are patients who find it generally difficult to swallow solid single unit dosage forms, including children and elderly people, or patients with certain types of diseases which make swallowing difficult.

The following examples are intended to further illustrate the invention, but not to limit its scope to the embodiments presented herein.

In all of the following examples, particle size measurements were performed by laser diffraction on a Malvern MasterSizer 2000 equipped with a Scirrocco 2000 dry-dispersing unit (Malvern Instruments, Herrenberg, Germany) using a Fraunhofer model for calculation. Each result quoted is the mean of three measurements. The particle sizes are stated as mean diameter of the volume distribution (D_{50%}).

For determining the dissolution time, the granules were added to water of 20°C in the stated concentration in a glass beaker placed on a magnetic stirrer adjusted to a constant rotation speed. The dissolved state was visually determined against a black background.

The bulk density was determined according to Ph. Eur. The pH was measured with an electronic pH-meter.

### Example 1: Aztreonam granules

This example describes the preparation of granules containing Aztreonam, an acidic drug substance which is nearly insoluble in water, and which possesses an intrinsic pH of about 1. In this case, the neutralization agent is the amino acid lysine.

100.0 g of lysine monohydrate were sieved through a 355 µm sieve to remove clumps. 82.1 g of the sieved lysine monohydrate were added to 112.5 g of Aztreonam in the 1l mixing-bowl of a Diosna P1-6 high shear mixer. The powders were blended for 3 minutes at a mixer speed of 500 rpm, with the chopper set at 0 rpm. Blending was interrupted every minute in order to remove powder from the bowl's walls.

After the blending was completed, 8 ml of purified water were slowly added to the mixture through a hole in the lid of the mixing bowl using a pump. During water addition, the mixer speed was kept at 500 rpm while the chopper speed was set to 1500 rpm. Mixing was performed for another 3 - 5 minutes. Thereafter, the wetted powder mixture was sieved through a 710 µm sieve and dried on trays at 40°C for 3 hours.

The resulting granules were analyzed and the properties were compared to those of the drug substance before granulation:

| | | |
|---|---|---|
| | **Drug substance** | **Granules** |
| D_{50%} (laser diffraction) | 67 µm | 321 µm |
| Bulk density | 0.49 g/ml | 0.58 g/ml |
| Aqueous solubility | < 0.1 % | > 10 % |
| pH of a 10% solution | - | 4.4 |

### Example 2: Aztreonam granules

Also this example describes the preparation of granules containing Aztreonam, an acidic drug substance which is nearly insoluble in water, and which possesses an intrinsic pH of about 1. In this case, the neutralization agent is sodium hydroxide.

17.8 g of sodium hydroxide were dissolved in 140.0 ml of ethanol (96 % v/v). 5.0 g of Aztreonam were weighed into a mortar. 10 ml of the ethanolic sodium hydroxide solution were then added to the Aztreonam under stirring. The resulting suspension was allowed to dry at room temperature (20 °C), until a slightly wet mass with good plasticity was obtained. This mass was passed through a sieve (710 µm mesh size) in order to obtain granules. The granules were dried on trays for three hours at 40°C. Finally, the granules were equalized by sieving through a 500 µm and a 250 µm sieve.

The following properties were found:

| | **Drug substance** | **Granules** |
|---|---|---|
| D_{50%} (laser diffraction) | 67 µm | 250-500 µm |
| Dissolution time (1 % solution) | - | 8.3 sec |
| Aqueous solubility | < 0.1 % | > 10 % |
| pH of a 10% solution | - | 6.6 |

### Example 3: Aztreonam granules

Example 3 describes the preparation of Aztreonam granules with enhanced dissolution behavior. The neutralization agent is sodium hydroxide.

17.8 g of sodium hydroxide were dissolved in 140.0 ml of ethanol (96 % v/v) containing 0.5 % (w/w) tyloxapol. 5.0 g of Aztreonam were weighed into a mortar. 10 ml of the ethanolic sodium hydroxide solution containing tyloxapol were added to the Aztreonam under stirring. The resulting suspension was allowed to dry at room temperature (20 °C), until a slightly wet mass with good plasticity was obtained. This mass was passes through a sieve (710 µm mesh size) in order to obtain granules. The granules were dried on trays for three hours at 40°C. Finally, the granules were equalized by sieving through a 500 µm and a 250 µm sieve.

It was found that, even with their content of tyloxapol, the granules possessed a sufficient degree of mechanical stability. Further properties were found as follows:

| | **Drug substance** | **Granules** |
|---|---|---|
| D_{50%} (laser diffraction) | 67 µm | 250-500 µm |
| Dissolution time (1 % solution) | - | 7.0 sec |
| Aqueous solubility | < 0.1 % | > 10 % |
| pH of a 10% solution | - | 6.7 |

Without wishing to be bound by a theory, it is believed that the dissolution time is reduced by more than 10% in comparison to the granules of example 2 because of the incorporation of the surfactant tyloxapol. As an alternative to the preparation process used herein, the surfactant can also be added to the drug prior to the granulation step. In this case, however, vigorous blending is necessary in order to coat the drug particles with a thin film of surfactant. Alternative surfactants include the polysorbates and vitamin E-TPGS.

### Example 4: Aztreonam granules

Also in this example, the preparation of Aztreonam granules with enhanced dissolution behavior is described, only that this effect is not achieved with a surfactant, but with the incorporation of a highly soluble excipient, glucose. The neutralization agent is sodium hydroxide.

53.3 g of sodium hydroxide were dissolved in 420.0 ml of ethanol (96 % v/v). 10.0 g of Aztreonam were weighed into a mortar. 1.0 g of glucose was added to the drug substance, and a homogenous powder mixture was generated. 20 ml of the ethanolic sodium hydroxide solution were then added to the powder mixture under stirring. The resulting suspension was allowed to dry at room temperature (20 °C), until a slightly wet mass with good plasticity was obtained. This mass was passed through a sieve (710 µm mesh size) in order to obtain granules containing glucose. The granules were dried on trays for three hours at 40°C. Finally, the granules were equalized by sieving through a 500 µm and a 250 µm sieve.

The properties were as follows:

| | **Drug substance** | **Granules** |
|---|---|---|
| D_{50%} (laser diffraction) | 67 µm | 250-500 µm |
| Dissolution time (1 % solution) | - | 7.3 sec |
| Aqueous solubility | < 0.1 % | > 10 % |
| pH of a 10% solution | - | 7.2 |

It is believed that it is due to the addition of glucose that the granules have a remarkably short dissolution time, probably facilitated by a rapid disintegration of the individual granule grains upon the dissolution of the glucose. Alternative highly-soluble excipients which may be used in the same way as glucose include lactose and mannitol.

### Example 5: Papaverine granules

This example describes the preparation of granules containing the drug substance papaverine, which is a base with a pK of 8.07, and which is nearly insoluble in water. In this case, the neutralization agent is citric acid.

2.5 g of papaverine were weighed into a mortar. 2.4 g of powdered citric acid were added and a homogenous powder mixture was generated. Purified water was added dropwise to the powder mixture under stirring until a slightly wet mass with good plasticity was formed. This mass was homogenized for 2 - 3 minutes. The mass was then granulated through a 710 µm sieve and dried on trays for 3 hours at 40°C.

The resulting granules were analyzed and the properties were compared to those of the drug substance before granulation:

| | **Drug substance** | **Granules** |
|---|---|---|
| D_{50%} (laser diffraction) | 28.5 µm | 330.5 µm |
| Bulk density | 0.41 g/ml | 0.51 g/ml |
| Aqueous solubility | Nearly insoluble | > 5 % |
| Dissolution time (5 % solution) | - | 98 sec |

### Example 6: Papaverine granules

In this example, papaverine granules are prepared using ethanol as a granulation liquid.

5.0 g of papaverine were weighed into a mortar. 4.8 g of powdered citric acid were added and a homogenous powder mixture was generated. Ethanol (99% v/v) was added dropwise to the powder mixture under stirring until a slightly wet mass with good plasticity was formed. This mass was homogenized for 2 - 3 minutes. The mass was then granulated through a 710 µm sieve and dried on trays for 3 hours at 40°C.

The properties were as follows:

| | **Drug substance** | **Granules** |
|---|---|---|
| D_{50%} (laser diffraction) | 28.5 µm | 290 µm |
| Bulk density | 0.41 g/ml | 0.59 g/ml |
| Aqueous solubility | Nearly insoluble | > 5 % |
| Dissolution time (5 % solution) | - | 117 sec |

### Example 7: Papaverine granules

This example describes the preparation of papaverine granules with enhanced dissolution behavior, which may be attributed to the presence of the surfactant tyloxapol. The neutralization agent is citric acid.

5.0 g of papaverine were weighed into a mortar. 1.0 g of tyloxapol was added to the drug substance. A homogenous mixture was formed through vigorous mixing, resulting in the coating of the drug particles with the surfactant. 4.8 g of powdered citric acid were added to the mixture of the drug substance and tyloxapol, and a homogenous powder mixture was generated. Ethanol (99% v/v) was added dropwise under stirring until a slightly wet mass with good plasticity was formed. This mass was homogenized for 2 - 3 minutes. The mass was then granulated through a 710 µm sieve and dried on trays for 3 hours at 40°C.

The properties were as follows:

| | **Drug substance** | **Granules** |
|---|---|---|
| D_{50%} (laser diffraction) | 28.5 µm | 619 µm |
| Bulk density | 0.41 g/ml | 0.56 g/ml |
| Aqueous solubility | Nearly insoluble | > 5 % |
| Dissolution time (5 % solution) | - | 73 sec |

Compared to the papaverine granules of example 6, the incorporation of tyloxapol resulted in a dramatic decrease in dissolution time, even though the granules of example 7 have a much smaller surface due to their larger particle size. The particle size of the resulting granules can be adjusted, however, by selecting the appropriate sieve, or by drying the mass prior to sieving.

## Claims

1. A process for preparing pharmaceutical granules which contain an active ingredient in the form of a salt, said process comprising the steps of:
(a) providing a powder containing the active ingredient as a free base or acid, and
(b) agglomerating the powder by adding a granulation liquid to form granules;
wherein step (b) is conducted in the presence of a neutralization agent capable of neutralizing the active ingredient, and for a sufficient amount of time to allow the active ingredient to become at least partially converted into a salt.

2. The process of claim 1, wherein step (b) is conducted for a sufficient amount of time and in the presence of a sufficient amount of neutralization agent to neutralize substantially all of the active ingredient contained in the powder.

3. The process of claim 1 or 2, wherein the free base or acid of the active ingredient contained in the powder has a water solubility at room temperature of less than 5 wt.-%.

4. The process of any of the preceding claims, wherein the salt formed by neutralizing the free base or acid of the active ingredient with the neutralization agent has a water solubility at room temperature which is at least 1 wt.-%.

5. The process of any of claims 1 to 3, wherein the salt formed by neutralizing the free base or acid of the active ingredient with the neutralization agent has a water solubility at room temperature which is at least twice as high as that of the free base or acid.

6. The process of any of the preceding claims, wherein the granulation liquid is an aqueous liquid.

7. The process of any of the preceding claims, wherein the granulation liquid comprises at least one organic solvent selected from alcohols, acetone and methylene chloride.

8. The process of any of the preceding claims, wherein the granulation liquid comprises at least one organic solvent selected from ethanol, methanol, isopropanol and mixtures thereof.

9. The process of any of the preceding claims, wherein the neutralization agent is provided as a component of the powder.

10. The process of any of claims 1 to 8, wherein the neutralization agent is provided as a component of the granulation liquid.

11. The process of any of the preceding claims, wherein step (b) is carried out in a mixer, high-shear mixer, fluid-bed granulator, or rotary granulator.

12. The process of any of the preceding claims, wherein the granulation liquid is added to the powder by spraying the liquid through a nozzle onto the powder.

13. Pharmaceutical granules containing an active ingredient in the form of a salt, said granules being obtainable by a process comprising the steps of:
(a) providing a powder containing the active ingredient as a free base or acid; and
(b) agglomerating the powder by adding a granulation liquid to form granules;
wherein step (b) is conducted in the presence of a neutralization agent capable of neutralizing the active ingredient and for a sufficient amount of time to allow the active ingredient to become at least partially converted into a salt.

14. The granules of claim 13, **characterized in that** they are substantially free of polymeric excipients such as polymeric binders and disintegrants.

15. The granules of claim 13 or 14, comprising one or more additional excipients, preferably selected from the group of bulking agents, fillers, binders, surfactants, stabilizers, preservatives, antioxidants, disintegrants, coloring agents, taste masking agents, sweeteners, flavors, release modifiers, plasticizers, and compression aids.

16. The granules of claim 15, comprising a surfactant selected from the group of tyloxapol, polysorbates, phospholipids, and vitamin E-TPGS.

17. The granules of any of claims 13 to 16, comprising an excipient with a water solubility at room temperature of at least 10 wt.-%.

18. The granules of any of claims 13 to 17, comprising an excipient selected from the group of sugars and sugar alcohols.

19. The granules of any of claims 13 to 18, **characterized in that** they are soluble in water or in a physiologically acceptable aqueous vehicle at room temperature to form a solution which is suitable for inhalation.

20. The granules of claim 19, **characterized in that** they are dissolvable in water or in a physiologically acceptable aqueous vehicle within less than 30 seconds at room temperature.

21. The granules of any of claims 13 to 20, comprising an active ingredient selected from the group of salbutamol, levalbuterol, formoterol, fenoterol, salmeterol, bambuterol, brocaterol, tiotropium, oxitropium, ipratropium, lidocaine, procaine, cystein, cromoglycinic acid, beclomethasone, triamcinolone, amoxicillin, ceftibuten, cefoxitin, aztrenonam, colistin, tobramycin, doxycycline, sildenafil, vardenafil, barbituric acid derivatives, benzodiazepines, morphine, codeine, salicylic acid, and their derivatives, conjugates, isomers, epimers, diastereomers, or racemic mixtures.

22. The granules of any of claims 13 to 21, having a weight-average particle size between 100 and 800 µm.

23. A pharmaceutical composition, comprising granules according to any of claims 13 to 22.

24. The pharmaceutical composition of claim 23, constituting a tablet prepared by compressing the granules and, optionally, further excipients.

25. The pharmaceutical composition of claim 23, constituting a hard capsule filled with the granules and, optionally, further excipients.

26. The use of pharmaceutical granules for the pulmonary delivery of an active ingredient.

27. The use of claim 26, wherein the pharmaceutical granules are substantially free of polymeric excipients, such as polymeric binders.

28. The use of claim 26 or 27, wherein the pharmaceutical granules are substantially free of insoluble excipients.

29. The use of any of claims 26 to 28, wherein the pharmaceutical granules contain an active ingredient in the form of a salt, and wherein the granules have been prepared by a process comprising the steps of:
(a) providing a powder containing the active ingredient as a free base or acid; and
(b) agglomerating the powder by adding a granulation liquid to form granules;
wherein step (b) is conducted in the presence of a neutralization agent capable of neutralizing the active ingredient and for a sufficient amount of time to allow the active ingredient to become at least partially converted into a salt.

30. The use of any of claims 26 to 29, wherein the granules are dissolved in an aqueous carrier to prepare a solution for inhalation.

31. The use of claim 30, wherein the solution for inhalation is aerosolized with a nebulizer.
